(19) Europäisches Patentamt
European Patent Office
Office européen des brevets

(11) **EP 4 729 043 A1**

(12) **EUROPEAN PATENT APPLICATION**

(43) Date of publication:
**22.04.2026 Bulletin 2026/17**

(21) Application number: **24306693.3**

(22) Date of filing: **15.10.2024**

(51) International Patent Classification (IPC):
*A61K 8/02* *(2006.01)* *A61K 8/26* *(2006.01)*
*A61K 8/27* *(2006.01)* *A61K 8/28* *(2006.01)*
*A61K 8/29* *(2006.01)* *A61Q 17/04* *(2006.01)*

(52) Cooperative Patent Classification (CPC):
**A61Q 17/04; A61K 8/0241; A61K 8/0287;**
**A61K 8/19; A61K 8/26; A61K 8/27; A61K 8/28;**
**A61K 8/29; C01G 9/02;** A61K 2800/412;
A61K 2800/413; A61K 2800/621; A61K 2800/651

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Nexdot**
**93230 Romainville (FR)**

(72) Inventors:
• **D'AMICO, Michele**
  **93230 Romainville (FR)**
• **LIN, Yu-Pu**
  **93230 Romainville (FR)**
• **DUBERTRET, Benoit**
  **93230 Romainville (FR)**

(74) Representative: **Icosa**
  **83 avenue Denfert-Rochereau**
  **75014 Paris (FR)**

(54) **PERSONAL CARE COMPOSITION COMPRISING COMPOSITE PARTICLES**

(57) The present invention relates to a personal care composition comprising composite particles having a mean size in a range from 100 nm to 10 μm, comprising (a) nanoparticles having a size of less than 50 nm along at least one dimension; and comprising at least one of zinc chalcogenide nanoparticles or titanium oxide nanoparticles; and (b) a metal oxide matrix in which the nanoparticles are embedded.

**FIG. 1**

**Description**

**FIELD OF INVENTION**

The present invention relates to personal care compositions comprising inorganic nanoparticles intended to absorb ultraviolet light.

**BACKGROUND**

Many ultraviolet screening agents are known in the personal care applications, for example inorganic ultraviolet screening agents also known as mineral screening agents, such as titanium dioxide ($TiO_2$) and zinc oxide (ZnO), and organic ultraviolet screening agents which usually comprise unsaturated cycles such as benzophenone derivatives and cinnamic acid derivatives. Such organic agents are suspected to cause skin sensibilization, health concern and induce environmental damages, which are drivers to limit or avoid their use.

Photoprotection using inorganic ultraviolet screening agents is a very important expectation of consumers seeking for sun protection. Many consumers consider mineral sunscreens to be safer for sensitive skin. $TiO_2$ and ZnO are the most common inorganic ultraviolet screening agents. However, the efficiency of $TiO_2$ and ZnO is limited, in particular in the UV-A wavelength range (320 nm to 380 nm). In addition, to achieve high protection (SPF values up to 50 for example), large amounts of ultraviolet screening agents are necessary, which induces substantial whitening effects and/or unpleasant sensations after application to the skin. Novel materials are thus sought which are capable of efficiently blocking UV rays (i.e., materials with a low UV ray transmission), in particular in the UVA range, and which have high transparency to visible light (*i.e.,* materials with a high transmission of rays between 380 nm and 780 nm). In other words, such materials should have a "steep" filtration front between the low transmittance region - ultraviolet - and the high transmittance region - visible light. In addition, these novel materials should not whiten after application.

Inorganic ultraviolet screening agents are usually particles with nanometric sizes. Indeed a nanometric size may lead to quantum effects with very "steep" filtration front. However, nanometric size potentially allows a transfer though the skin with potential unwanted health effects. The compromise between small size - to avoid light diffusion and whitening effect, and to design appropriate light absorption properties - and large size - to ensure safety for consumers - has not found a satisfactory solution yet.

One of the objects of the present disclosure is to provide a material for screening out ultraviolet rays, which is intrinsically capable of screening out only ultraviolet rays but having a large size.

This objective has been reached with composite particles comprising ultraviolet screening inorganic nanoparticles. The composite particles can have a size tailored to prevent transfer through skin, while comprising nanoparticles with very small size leading to adequate absorption of ultraviolet rays.

**SUMMARY**

This disclosure thus relates to a personal care composition comprising

- a cosmetically acceptable medium;
- composite particles having a mean size in a range from 100 nm to 10 μm, said composite particles comprising:

    o nanoparticles having a size of less than 50 nm along at least one dimension; and comprising at least one of zinc chalcogenide nanoparticles or titanium oxide nanoparticles; and
    o a metal oxide matrix in which the nanoparticles are embedded.

In a first configuration, the nanoparticles comprise zinc chalcogenide nanoparticles comprising homostructured zinc chalcogenide nanoparticles having a composition defined by formula ZnX (I); where X is selected from sulfur, selenium, tellurium or mixtures thereof, preferably X is a mixture comprising more than 60 at% of selenium.

In an embodiment of the first configuration, the nanoparticles comprise zinc chalcogenide nanoparticles comprising heterostructured zinc chalcogenide nanoparticles having a composition defined by formula $ZnX_1/ZnX_2$ (II), where $X_1$ is selected from oxygen, sulfur, selenium, tellurium and mixtures thereof; $X_2$ is selected from oxygen, sulfur, selenium, tellurium and mixtures thereof; and $X_1$ and $X_2$ are different.

In an embodiment of the first configuration, the zinc chalcogenide nanoparticles have local maximum absorbance of highest wavelength in a range from 350 nm to 450 nm.

In an embodiment of the first configuration, the personal care composition further comprises composite particles having a mean size in a range from 100 nm to 10 μm, said composite particles comprising titania nanoparticles embedded in the metal oxide matrix, and said titania nanoparticles having a size of less than 50 nm along at least one dimension, preferably less than 20 nm along at least one dimension.

In an embodiment of the first configuration, the personal care composition further comprises composite particles having a mean size in a range from 100 nm to 10 µm, said composite particles comprising zinc oxide nanoparticles embedded in the metal oxide matrix, and said zinc oxide nanoparticles having a size of less than 50 nm along at least one dimension, preferably less than 35 nm along at least one dimension.

In a second configuration, the composite particles comprise:

- zinc chalcogenide nanoparticles that are not zinc oxide nanoparticles and having a size of less than 20 nm along at least one dimension; and
- at least one of:

  o titania nanoparticles having a size of less than 20 nm along at least one dimension ;
  o zinc oxide nanoparticles having a size of less than 35 nm along at least one dimension.

In an embodiment, the atomic ratio defined by the amount of zinc element in zinc chalcogenide nanoparticles that are not zinc oxide nanoparticles divided by the amount of zinc and titanium elements in zinc oxide nanoparticles and titanium oxide nanoparticles is in a range from 0.2 to 5.

In an embodiment, the loading charge of the nanoparticles in the composite particle is at least 1%, preferably at least 2.5%, more preferably at least 5%, said loading charge being the mass ratio between the mass of nanoparticles comprised in a composite particle and the mass of said composite particle.

In an embodiment, the loading charge of the zinc chalcogenide nanoparticles that are not zinc oxide nanoparticles in the composite particle is at least 1%, preferably at least 2.5%, more preferably at least 5%, said loading charge being the mass ratio between the mass of zinc chalcogenide nanoparticles that are not zinc oxide nanoparticles comprised in a composite particle and the mass of said composite particle.

In a third configuration, the composite particles comprise:

- titania nanoparticles having a size of less than 20 nm along at least one dimension ; and
- zinc oxide nanoparticles having a size of less than 35 nm along at least one dimension.

In an embodiment of the third configuration, the loading charge of the nanoparticles in the composite particle is at least 1%, preferably at least 2.5%, more preferably at least 5%, said loading charge being the mass ratio between the mass of nanoparticles comprised in a composite particle and the mass of said composite particle.

In an embodiment - for any configuration - the metal oxide matrix is selected from the group consisting of $SiO_2$, $Al_2O_3$, $ZrO_2$, $HfO_2$, $GeO_2$, $SnO_2$, ZnO or a mixture thereof, preferably, the metal oxide matrix is alumina, $Al_yZr_zO$ with

$$\frac{3}{2}y + 2z = 1 \text{ or } Si_pZr_qO \text{ with } p + q = \frac{1}{2}.$$

In an embodiment - for any configuration - the personal care composition substantially absorbs ultraviolet light.

In an embodiment - for any configuration - the refractive index of composite particles and the refractive index of cosmetically-acceptable medium are matched, preferably the difference between the refractive index of composite particles and the refractive index of cosmetically-acceptable medium is less than 0.7, preferably less than 0.5, more preferably less than 0.4. In some configurations, the difference between the refractive index of composite particles and the refractive index of cosmetically-acceptable medium may be less than 0.25, ideally less than 0.1.

In an embodiment - for any configuration - the personal care composition is a hair conditioner, a moisturizer, a suntan lotion, a lipstick, or a hair gel.

## DEFINITIONS

In the present invention, the following terms have the following meanings:

**"Absorbance"** is the decimal logarithm of ratio $I_0/I$, where $I_0$ is the intensity of light incident on a sample and I is the intensity of light transmitted through said sample. In this disclosure, absorbance of liquid dispersions is measured for a 2-millimeter-thick sample with correction of the absorbance of the pure solvent - blank solvent. Absorbance is measured for wavelengths in ultraviolet and visible range from 280 nm to 780 nm.

**"at%"** refers to the atomic percentage of an element in a compound. In this disclosure, the atomic percentage for a metallic element of a metal oxide matrix is given without considering oxygen. For instance, $Al_2O_3$ contains 100 at% of aluminum as no other metallic element is present in the metal oxide.

**"Chalcogenides"** refers to an element form column $VI_B$ of the periodic table of elements except Polonium - for obvious toxicity issues - namely Oxygen (O), Sulphur (S), Selenium (Se) and Tellurium (Te).

**"Core-shell"** refers to heterogeneous nanostructure comprising an inner part: the core, overcoated on its surface by a layer of at least one atom thick material different from the core: the shell. Core-shell structures are noted as follows: core material-shell material. Core-shell nanostructure also include nanoparticles in which the central part: the core, is embedded - or encapsulated - by a layer of material disposed on the core: the shell, said shell having a gradient of composition from the core to the outside of the shell. In this case, the composition of the nanoparticle is changing smoothly - for instance continuously - from the core composition to the outside composition of the shell. There is no precise boundary between core and shell but properties in centre of the core are different from properties on the outer boundary of shell. In addition, core and shell may have different shapes, for instance a dot - a nanosphere or a nanocube or any other nanocluster - is provided as a core and shell is grown laterally around the core, yielding an heterostructure with shape of a nanoplate but comprising a dot inside the nanoplate.

**"Embedded"** or **"Encapsulated"** refers to a state in which a material - an encapsulating material - coats, surrounds, embeds, contains, comprises, wraps, packs, or encloses a plurality of particles, which may be nanoparticles or composite particles.

**"Loading charge"** refers to the mass ratio between the mass of particles comprised in a formulation and the mass of said formulation. For the sake of clarity, 10g of particles mixed with 90g of a matrix defines a loading charge of 10%.

**"Mean size"** refers to a size of a population of particles, obtained by a mathematical mean of sizes of each individual particle of the population. Practically, the mean size may be determined by electronic microscopy: size of each particle visible in the microscopy is evaluated by fitting each particle with a circle whose diameter defines the size of the particle, then computing the mean of all individual sizes to obtain the mean size. Other methods, such as light scattering may be used to determine indirectly the mean size of the population of particles. Experimentally, particles are always obtained in the form of population of particles. By extension in this disclosure, the mean size of a particle is the mean size of the population of particles which has been synthesized. For the sake of clarity, a particle having a mean size between 100 nm and 250 nm is a particle representative of a population of particles having a mean size between 100 nm and 250 nm.

**"Nanometric size"** refers to a size of matter in which quantum effects appear due to confinement. For semi-conductive nanoparticles, nanometric size has to be defined with the average Bohr radius of an electron/hole pair. Confinement is effective for size in at least one dimension of nanoplates below 10 nm, preferably below 5 nm. Confinement is effective for section of nanorods below 100 nm$^2$, preferably below 50 nm$^2$. Confinement is effective for diameter of nanospheres below 20 nm, preferably below 15 nm, more preferably below 10 nm.

**"Nanoparticle"** refers to a particle having a size in at least one of its dimensions below 100 nm. For a nanosphere, diameter should be below 100 nm. For a nanoplate, thickness should be below 100 nm. For a nanorod, diameter should be below 100 nm.

**"Nanoplate"** refers to a two-dimensional shaped nanoparticle, wherein the smallest dimension of said nanoplate - the thickness - is smaller than the largest dimension of said nanoplate by a factor (aspect ratio) of at least 1.5, at least 2, at least 2.5, at least 3, at least 3.5, at least 4, at least 4.5, at least 5, at least 5.5, at least 6, at least 6.5, at least 7, at least 7.5, at least 8, at least 8.5, at least 9, at least 9.5 or at least 10. Nanoplates include nanosheets, nanoribbons or nanodisks.

**"Nanorod"** refers to a one-dimensional shaped nanoparticle, wherein the average dimension of the section of said nanorod is smaller than the length of the nanorod by a factor (aspect ratio) of at least 1.5, at least 2, at least 2.5, at least 3, at least 3.5, at least 4, at least 4.5, at least 5, at least 5.5, at least 6, at least 6.5, at least 7, at least 7.5, at least 8, at least 8.5, at least 9, at least 9.5 or at least 10. Nanorods include nanowires and nanorings.

**"Nanosphere"** refers to a three-dimensional shaped nanoparticle having similar dimensions in the three directions. Nanospheres also include nanocubes or nanodots.

**"Ultraviolet light":** refers to electromagnetic radiations having a wavelength comprised between 280 nm and 380 nm, *i.e.,* the **ultraviolet range.** In this disclosure UV-C light having wavelength below 280 nm is not considered.

**"Visible light"** refers to electromagnetic radiations having a wavelength comprised between 380 nm and 780 nm.

**"%wt"** refers to the weight fraction of a component in a blend or a formulation.

## DETAILED DESCRIPTION

This disclosure relates to a personal care composition comprising a cosmetically acceptable medium and composite particles.

*Cosmetically acceptable medium*

In this disclosure, a cosmetically acceptable medium is a medium which may be applied to skin or at least one keratinous substance. In one embodiment, the cosmetically acceptable medium is chosen from at least one solvent, water and mixtures thereof. Non-limiting examples of the at least one solvent include organic solvents. Non-limiting examples of the cosmetically acceptable medium include aqueous-alcoholic mixtures such as mixtures comprising at least one C1-C5 monoalcohol. Non-limiting examples of organic solvents include: ketones which are liquid at room temperature (such as methyl ethyl ketone, methyl isobutyl ketone, diisobutyl ketone, isophorone, cyclohexanone, and acetone); alcohols which are liquid at room temperature (such as ethanol, isopropanol, diacetone alcohol, 2-butoxyethanol, and cyclohexanol); glycols which are liquid at room temperature (such as ethylene glycol, propylene glycol, pentylene glycol, and glycerol); propylene glycol ethers which are liquid at room temperature (such as propylene glycol monomethyl ether, propylene glycol monomethyl ether acetate, and dipropylene glycol mono-n-butyl ether); short-chain esters which comprise from 3 to 8 carbon atoms (such as ethyl acetate, methyl acetate, propyl acetate, n-butyl acetate, and isopentyl acetate) and are liquid at room temperature; ethers which are liquid at room temperature (such as diethyl ether, dimethyl ether, and dichlorodiethyl ether); alkanes which are liquid at room temperature (such as decane, heptane, dodecane, and cyclohexane); cyclic aromatic compounds which are liquid at room temperature (such as toluene and xylene); and aldehydes which are liquid at room temperature (such as benzaldehyde and acetaldehyde). Other suitable at least one solvent include Alkyl salicylate, Butyloctyl salicylate, C10-15 alkyl lactate, C12-15 Alkyl benzoate, C12-15 Triethoxy alkyl benzoate, Cocoglycerides, Dipropylene glycol benzoate, Glyceryl stearate laurethyl 23, Isocetyl salicylate, Isodecyl isononanoate, Isodecyl salicylate, Isononyl isononanoate, Isostearyl benzoate, Isotridecyl isononanoate, Isotridecyl isononanoate, Methyl gluceth-20 benzoate, Octyldodecyl benzoate, PEG-20 stearate, Poloxamer 105 benzoate, Poloxamer 182 dibenzoate, PPG-15 stearyl ether benzoate, Tridecyl salicylate.

The skilled artisan in personal care formulation will recognize which cosmetically-acceptable medium, and therefore the at least one solvent, will be chosen according to the desired characteristics and application of the inventive composition. For example, certain solvents are known in the art to be especially suitable for certain applications, such as sun protection.

According to an embodiment, the cosmetically acceptable medium may be present in an amount generally ranging from 30% to 99% by weight, relative to the total weight of the composition, such as from 60% to 90%.

In this disclosure, the composite particles have a mean size in a range from 100 nm to 10 $\mu$m. This range of size has proven particularly suitable to prevent transfer through the skin. Preferably, the composite particles have a mean size less than 5 $\mu$m, more preferably less than 1 $\mu$m, thereby maintaining high transparency - due to low light scattering. In a preferred embodiment, the composite particles have a mean size in a range from 125 nm to 750 nm, more preferably in a range from 150 nm to 500 nm.

A key point to maintain high transparency of the personal care composition after application is the control of light scattering. Indeed, the white aspect of composition comprising particles is linked to the scattering of light induced by the refractive index difference between particles and cosmetically-acceptable medium. The low scattering effect is a result of composite particles size and composite particles refractive index: the lower the refractive index, the larger may be the composite particles. The refractive index of the composite particle may be adjusted by the loading charge of nanoparticles - whose refractive index is usually greater than the metal oxide matrix used. The refractive index of the composite particle may be also adjusted with some internal porosity of the metal oxide matrix. It has to be noted that such porosity may be open, *i.e.,* accessible from outside and corresponding to a high specific surface as measured according to BET methods. On the contrary, such porosity may be closed, *i.e.,* not accessible from outside and corresponding to a low specific surface as measured according to BET methods. Without being bound by theory, it is believed that composite particles with closed porosity comprise an external layer that is not permeable to gas. The refractive index of the composite particle may be last adjusted by the composition of the metal oxide matrix: using silicon in the metal oxide matrix leads to a lower refractive index for instance. In an embodiment, refractive index of composite particles and refractive index of cosmetically-acceptable medium are matched to prevent light scattering and whitening effect. Preferably, the difference between the refractive index of composite particles and the refractive index of cosmetically-acceptable medium is less than 0.7, more preferably less than 0.5, ideally less than 0.4.

In addition, composite particles below micron size show improved dispersibility in order to present a homogeneous distribution of composite particle - hence sun protection - in the personal care composition.

Last, the nanoparticles being dispersed in the metal oxide matrix, they do not form aggregates. Therefore, the unique optical properties of nanosized particles are maintained, but in the form of large composite particles preventing transfer through the skin.

*Nanoparticles*

In this disclosure, the composite particles comprise nanoparticles, said nanoparticles having a size of less than 50 nm along at least one dimension. The nanoparticles have preferably a size of less than 35 nm along at least one dimension, more preferably a size of less than 20 nm along at least one dimension. Such nanometric size leads to quantum effects in the nanoparticles associated with specific light absorption properties.

Said nanoparticles comprise at least one of zinc chalcogenide nanoparticles or titanium oxide nanoparticles. Indeed, these materials present interesting absorption properties in ultraviolet range.

*Metal oxide matrix*

Last, the composite particles comprise a metal oxide matrix in which the nanoparticles are embedded. In the present disclosure, the composition of the matrix is theorical, and the atomic ratios should be understood as an aimed composition, provided that the final composition may comprise up to 10at% of impurities. The impurities may be additional elements that may be included in the composition. These impurities may be organic moieties coming from precursors of the metal oxide matrix. A matrix comprising less than 10 wt% of impurities, preferably less than 5 wt% of impurities is considered pure.

This structure of composite particles allows having nanoparticles with specific light absorption properties associated to a size below 50 nm available in a personal care composition in an object of larger size. Thus, the tradeoff between low size for optical properties and large size for skin transfer prevention is met.

Said metal oxide matrixes may be obtained by reaction of metal oxide precursors, in particular metal alkoxides or organometallics, as disclosed in WO2018/220165 for instance.

Suitable alkoxides are silicon alkoxides, aluminium alkoxides, zirconium alkoxides, titanium alkoxides and optionally other metal alkoxides. Said alkoxides may be used alone, yielding a pure matrix. Said alkoxides may be used in mixtures, yielding mixed matrixes.

Preferred silicon alkoxides are tetramethoxysilane $Si(OCH_3)_4$ (commonly called methyl silicate) and tetraethoxysilane $Si(OC_2H_S)_4$ (commonly called ethyl silicate) or methyltriethoxysilane.

Preferred aluminum alkoxides are aluminum tert-butoxyde.

Preferred zirconium alkoxides are zirconium tetra propoxide $Zr(OC_3H_7)_4$, for instance as a solution in 1-propanol.

Preferred titanium alkoxides are titanium tetraisopropoxide $Ti(OisoC_3H_7)_4.$, titanium tetraethoxide or titanium tetrabutoxide.

These alkoxides may be used alone, leading to pure matrix such as $SiO_2$, $Al_2O_3$, $ZrO_2$, $TiO_2$ or $HfO_2$; or in mixtures, leading to mixed matrixes such as SiZrO or $Al_2ZrO_5$ for instance.

Suitable organometallics are metallocenes; metal amidinates; metal alkyl halides; metal alkyls such as for example $Hf[NMe_2]_4$, $In[N(SiMe_3)_2]_3$, $Sn(NMe_2)_2$, $Sn[N(SiMe_3)_2]_2$, $Zn[N(SiMe_3)_2]_2$ or $Zn[(NiBu_2)_2]_2$, zinc diethylthiocarbamate, trimethyl aluminium, triisobutylaluminum, trioctylaluminum, triphenylaluminum, dimethyl aluminium, trimethyl zinc, dimethyl zinc, diethylzinc, $Zn[(N(TMS)_2]_2$, $Zn[(CF_3SO_2)_2N]_2$, $Zn(Ph)_2$, $Zn(C_6F_5)_2$, $Zn(TMHD)_2$ ($\beta$-diketonate), $Hf[C_5H_4(CH_3)]_2(CH_3)_2$, $HfCH_3(OCH_3)[C_5H_4(CH_3)]_2$, $[[(CH_3)_3Si]_2N]_2HfCl_2$, $(C_5H_5)_2Hf(CH_3)_2$, $[(CH_2CH_3)N]_4Hf$, $[(CH_3)_2N]_4Hf$, $[(CH_3)_2N]_4Hf$, $[(CH_3)(C_2H_5)N]_4Hf$, $[(CH_3)(C_2H_5)N]_4Hf$, 2,2',6,6'-tetramethyl-3,5-heptanedione zirconium $(Zr(THD)_4)$, $C_{10}H_{12}Zr$, $Zr(CH_3C_5H_4)_2CH_3OCH_3$, $C_{22}H_{36}Zr$, $[(C_2H_5)_2N]_4Zr$, $[(CH_3)_2N]_4Zr$, $[(CH_3)_2N]_4Zr$, $Zr(NCH_3C_2H_5)_4$, $Zr(NCH_3C_2H_5)_4$, $Ci_8H_{32}O_6Zr$, $Zr(C_8H_{15}O_2)_4$, $Zr(OCC(CH_3)_3CHCOC(CH_3)_3)_4$, or a mixture thereof. These organometallics may be used alone, leading to pure matrixes such as $HfO_2$, $ZnO$, $Al_2O_3$ or $ZrO_2$; or in mixtures, leading to mixed matrixes such as $Al_2ZrO_5$ for instance.

*Zinc chalcogenides nanoparticles*

In this disclosure, suitable zinc chalcogenide nanoparticles may be selected from semi-conductive nanoparticles known as quantum dots, which may have various shape and structure.

*Quantum dots shape*

In one embodiment, quantum dots may have different shapes, provided that they present a nanometric size leading to quantum confinement in the nanoparticle.

Quantum dots may have nanometric sizes in three dimensions, allowing quantum confinement in all three spatial dimensions. Such quantum dots are for instance nanocubes or nanospheres.

Quantum dots may have a nanometric sizes in two dimensions, the third dimension being larger: quantum confinement is in two spatial dimensions. Such quantum dots are for instance nanorods, nanowires or nanorings.

Quantum dots may have a nanometric size in one dimension, the other dimensions being larger: quantum confinement is in one spatial dimension only. Such quantum dots are for instance nanoplates, nanosheets, nanoribbons or nanodisks. Nanoplates are especially interesting in this disclosure because absorption cross section - *i.e.,* efficiency to capture a photon of incident light on the quantum dot - is ten times higher than a nanosphere having the same composition and structure emitting at the same wavelength. This higher cross section improves significantly ultraviolet light absorption.

The exact shape of quantum dots defines confinement properties, then electronic and optical properties.

*Quantum dots structure*

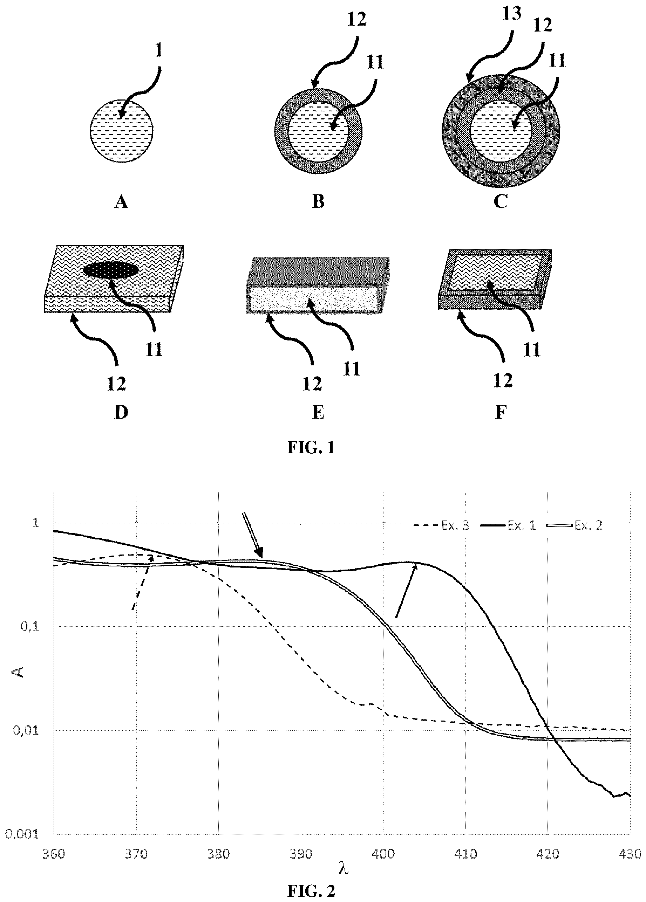

In an embodiment, quantum dots are homostructures. By homostructure, it is meant that the quantum dot is homogenous and has the same local composition in all its volume. A homogeneous spherical quantum dot is illustrated in figure 1A.

In an alternative embodiment, quantum dots are heterostructures. By heterostructure, it is meant that the quantum dot is comprised of several sub-volumes, each sub-volume having a different composition from neighbouring sub-volumes. In a particular embodiment, all sub-volumes have a composition defined by formula (II) disclosed above, with different parameters, *i.e.,* elemental composition and stoichiometry.

Example of heterostructure are core/shell nanoparticles, the core (11) having any shape disclosed above. A shell (12) is a layer covering totally or partially the core. A particular example of core/shell heterostructure is a multi-layered structure comprising a core (11) and several successive shells (12, 13). For convenience, these multi-layered heterostructures are named core/shell hereafter. Core (11) and shell (12,13) may have the same shape - sphere in sphere for example - or not - sphere in plate for instance. A core/shell spherical nanoparticle is illustrated in figure 1B. A core/shell/-shell spherical nanoparticle is illustrated in figure 1C. A sphere in plate nanoparticle is illustrated in figure 1D - also named a dot in plate. A core/shell nanoplate is illustrated in figure 1E.

Another example of heterostructure are core/crown nanoparticles, the core having any shape disclosed above. A crown is a band of material disposed on the periphery of the core. This heterostructure is particularly useful with cores being nanoplates and crown disposed on the edges of the nanoplate. A core/crown nanoplate is illustrated in figure 1F.

These heterostructure may have a gradient of composition from the core to the outside of the shell so that there is no precise boundary between core and shell but properties in centre of the core are different from properties on the outer boundary of shell.

According to a first embodiment, the nanoparticles comprise zinc chalcogenide nanoparticles comprise homostructured zinc chalcogenide nanoparticles having a composition defined by formula $ZnX$ (I), where X is selected from the group consisting of sulfur, selenium, tellurium and mixtures thereof.

The following nanoparticles - II-VI type - are especially suitable: $ZnS$, $ZnSe$, $ZnTe$, $ZnSe_xS_{(1-x)}$, $ZnSe_xTe_{(1-x)}$ where x is a rational number between 0 (excluded) and 1 (excluded). Here, x is preferably a rational number between 0.6 and 1 (excluded), corresponding to X being a mixture comprising more than 60 at% of selenium.

In an embodiment, the size of homostructured zinc chalcogenide nanoparticles is less than 20 nm along at least one dimension. Such small sizes are associated to more efficient quantum effects - confinement - and provide improved light absorption properties.

In addition to homostructured zinc chalcogenide nanoparticles, or as an alternative, the zinc chalcogenide nanoparticles comprise heterostructured zinc chalcogenide nanoparticles having a composition defined by formula $ZnX_1/ZnX_2$ (II), where $X_1$ is selected from oxygen, sulfur, selenium, tellurium and mixtures thereof, $X_2$ is selected from oxygen, sulfur, selenium, tellurium and mixtures thereof; and $X_1$ and $X_2$ are different.

Any type of core/shell, core/crown or dot in plate corresponds to these heterostructured zinc chalcogenide nanoparticles. The following nanoparticles - II-VI type - are especially suitable:

- $ZnSe/ZnS$, $ZnSe/ZnSe_xS_{(1-x)}$, $ZnSe/ZnO$, $ZnTe/ZnSe_xS_{(1-x)}$, $ZnTe/ZnO$,
- $ZnO/ZnS$, $ZnO/ZnSe$, $ZnO/ZnTe$, $ZnO/ZnSe_yS_{(1-y)}$,
- $ZnSe_xS_{(1-x)}/ZnS$, $ZnSe_xS_{(1-x)}/ZnSe$, $ZnSe_xS_{(1-x)}/ZnO$, $ZnSe_xS_{(1-x)}/ZnSe_yS_{(1-y)}$, $ZnSe_xTe_{(1-x)}/ZnS$, $ZnSe_xTe_{(1-x)}/ZnSe$, $ZnSe_xTe_{(1-x)}/ZnO$, $ZnSe_xTe_{(1-x)}/ZnSe_xS_{(1-y)}$,
- $ZnSe_xS_{(1-x)}/ZnS/ZnSe$, $ZnSe_xS_{(1-x)}/ZnS/ZnSe_yS_{(1-y)}$,

where x and y are rational numbers between 0 (excluded) and 1 (excluded). Here, x is preferably a rational number between 0.6 and 1 (excluded), corresponding to $X_1$ being a mixture comprising more than 60 at% of selenium.

In an embodiment, the size of heterostructured zinc chalcogenide nanoparticles is less than 20 nm along at least one dimension. Such small sizes are associated to more efficient quantum effects - confinement - and provide improved light absorption properties.

In particular, the zinc chalcogenide nanoparticles of formula (I) or (II) have local maximum absorbance of highest wavelength in a range from 350 nm to 450 nm. These local maxima are shown on figure 2 by arrows.

According to a specific configuration of the first embodiment, the personal care composition further comprises composite particles having a mean size in a range from 100 nm to 10 $\mu$m, said composite particles comprising titania nanoparticles having a size of less than 50 nm along at least one dimension, embedded in a metal oxide matrix. Titania nanoparticles having a size of less than 20 nm along at least one dimension are preferred. Embedding titania particles in a metal oxide matrix - not titania - also prevent the photoactivation of titania potentially generating free radicals in the personal care composition. The combination of titania nanoparticles having a strong absorption in the UV-B range with zinc chalcogenide nanoparticles of formula (I) or (II) having a strong absorption in the UV-A range provides a very efficient protection over all UV rays naturally occurring.

According to a specific configuration of the first embodiment, the personal care composition further comprises composite particles having a mean size in a range from 100 nm to 10 $\mu$m, said composite particles comprising zinc oxide nanoparticles having a size of less than 50 nm along at least one dimension, embedded in a metal oxide matrix. Zinc oxide nanoparticles having a size of less than 35 nm along at least one dimension are preferred. The combination of zinc oxide nanoparticles having a strong absorption in the UV-B range with zinc chalcogenide nanoparticles of formula (I) or (II) having a strong absorption in the UV-A range provides a very efficient protection over all UV rays naturally occurring.

In a very specific configuration of the first embodiment, the personal care composition comprises composite particles having a mean size in a range from 100 nm to 10 $\mu$m, said composite particles comprising zinc chalcogenide nanoparticles - of formula (I) or (II) - titania nanoparticles and zinc oxide nanoparticles.

Said combinations open ways to personal care composition devoid of organic UV-absorbers., *i.e.,* comprising less than 5 wt%, preferably less than 2 wt% of organic UV-absorbers.

According to a second embodiment, the composite particles comprise:

- zinc chalcogenide nanoparticles that are not zinc oxide nanoparticles and having a size of less than 20 nm along at least one dimension; and
- at least one of:

  o titania nanoparticles having a size of less than 20 nm along at least one dimension ;
  o zinc oxide nanoparticles having a size of less than 35 nm along at least one dimension.

In particular, the zinc chalcogenide nanoparticles of formula (I) or (II) of the first embodiment are not zinc oxide nanoparticles and are suitable for this second embodiment.

Composite particles comprising simultaneously at least two types of nanoparticles - as compared to a mixture of composite particles, each composite particle comprising a single type of nanoparticles - allow defining a single ingredient for personal care formulation with a specific UV absorption spectrum designed with the type and relative proportions of zinc chalcogenide nanoparticles on one hand, zinc oxide and/or titania nanoparticles on the other hand. Said combinations also leads to personal care composition devoid of organic UV-absorbers., *i.e.,* comprising less than 5 wt%, more preferably less than 2 wt% of organic UV-absorbers.

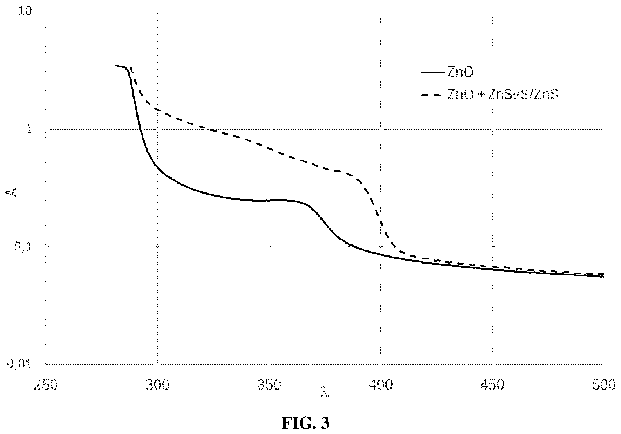

In embodiments where composite particles comprise zinc chalcogenide nanoparticles that are not zinc oxide nanoparticles - typically corresponding to formula (I) or (II) - and titania particles, the atomic ratio defined by the amount of zinc element in zinc chalcogenide nanoparticles that are not zinc oxide nanoparticles divided by the amount of zinc and titanium elements in zinc oxide nanoparticles and titanium oxide nanoparticles is preferably in a range from 0.2 to 5. This ratio leads to an optimization of the amount of nanoparticles to obtain an absorption spectrum with good absorption in both UVA and UVB ranges. In other words, the relatively low absorption of ZnO or $TiO_2$ in UVA range is compensated by zinc chalcogenides particles: less ZnO and $TiO_2$ is required globally in the personal care composition. This effect is clearly visible on figure 3. Absorbance of a first composition comprising ZnO nanoparticles - 2.3 wt% in the composition - embedded in alumina matrix is shown in solid line. A second composition comprising zinc chalcogenide nanoparticles of example 2 - 11 wt% in the composition - and ZnO nanoparticles - 2.3 wt% in the composition - embedded in alumina matrix is shown in dotted line. Zinc chalcogenide nanoparticles improve drastically the absorbance in UVA range, especially in the 360 nm-400 nm range, without imparting significantly the transparency of the personal care composition. The atomic ratio in the second composition of zinc element in zinc chalcogenide nanoparticles that are not zinc oxide nanoparticles divided by the amount of zinc element in zinc oxide nanoparticles is roughly 3.

In an embodiment - compatible with both first and second embodiment - the loading charge of the nanoparticles in the composite particle is at least 1%, preferably at least 2.5%, more preferably at least 5%.

In a specific configuration of the first embodiment, the loading charge of the zinc chalcogenide nanoparticles that are not zinc oxide nanoparticles in the composite particle is at least 1%, preferably at least 2.5%, more preferably at least 5%.

A high loading charge of zinc chalcogenide nanoparticles is preferred because UV-rays are absorbed by these zinc chalcogenide nanoparticles and the final efficiency of the personal care formulation is linked to the weight fraction of zinc chalcogenide nanoparticles, whatever the weight fraction of metal oxide matrix. A high loading charge also enables reduction of mean size of composite particles, thus reducing whitening effect and light scattering effect.

According to a third embodiment, the composite particles comprise titania nanoparticles having a size of less than 20 nm along at least one dimension; and zinc oxide nanoparticles having a size of less than 35 nm along at least one dimension. In this embodiment, the loading charge of the nanoparticles in the composite particle is at least 1%, preferably at least 2.5%, more preferably at least 5%.

In an embodiment - compatible with the first, second or third embodiment - the metal oxide matrix is selected from the group consisting of $SiO_2$, $Al_2O_3$, $ZrO_2$, $HfO_2$, $GeO_2$, $SnO_2$, or a mixture thereof.

It has to be noted that metal oxide matrix may be also ZnO - pure or in mixture with the other elements listed hereabove - with the proviso that pure ZnO is not suitable for composite particles comprising ZnO nanoparticles. Indeed, the composition of nanoparticles is essentially different from the composition of metal oxide matrix.

In an embodiment, the metal oxide matrix does not consist of pure silica - $SiO_2$.

Preferred metal oxide matrix are

- Alumina $Al_2O_3$;
- mixed oxide of Aluminium and Zirconium having the formula $Al_xZr_yM_zO$ where M represents one or more metal elements selected from the group of Si, Ti, Hf, Ge, Sn, Zn, or mixtures thereof; x is in the range from 0 to 2/3; y is in the range from 0 to 1/2; and z is in the range from 0 to 2/5. When z is null - which is a preferred embodiment - the relation between x and y is $(3/2x+2y)=1$.
- mixed oxide of Silicon and Zirconium having the formula $Si_pZr_qM_zO$ where M represents one or more metal elements selected from the group of Al, Ti, Hf, Ge, Sn, Zn, or mixtures thereof; p is in the range from 0 to 1/2; q is in the range from 0 to 1/2; and z is in the range from 0 to 2/5. When z is null - which is a preferred embodiment - the relation between p and q is $(p+q)=1/2$.

Preferably, the personal care composition disclosed herein absorbs substantially ultraviolet light. By substantially, it is meant that the personal care composition has a Sun Protection factor (SPF) greater than 5, preferably greater than 10, more preferably greater than 20.

Preferably, the personal care composition disclosed herein is a hair conditioner, a hair gel, a moisturizer, a suntan lotion, a sun protection lotion or a lipstick.

This disclosure also relates to a method of protecting at least a portion of a body against ultraviolet radiation, the method comprising applying a personal care composition as disclosed hereabove to at least a portion of the body.

## BRIEF DESCRIPTION OF THE DRAWINGS

**Figure 1A-1F** illustrates various nanoparticles (1) with homostructure (A) or heterostructures: spherical core/shell (B), spherical core/shell/shell (C), dot in plate (D), nanoplate core/shell (E) and nanoplate core/crown (F).

**Figure 2** is a graph showing absorbance A (Vertical axis, logarithmic scale, arbitrary unit) of various particles as a function of light wavelength ($\lambda$, in nm). Solid line corresponds to Example 1 using Zinc chalcogenide core-shell nanoparticles. Double line corresponds to Example 2 using Zinc chalcogenide core-shell nanoparticles. Dotted line corresponds to Example 3 using ZnSe nanoparticles.

**Figure 3** is a graph showing absorbance A (Vertical axis, logarithmic scale, arbitrary unit) as a function of light wavelength ($\lambda$, in nm) of a personal care composition comprising ZnO nanoparticles embedded in an alumina matrix (solid line) and another personal care composition comprising ZnO and ZnSeS/ZnS nanoparticles embedded in an alumina matrix (dotted line).

## EXAMPLES

The present invention is further illustrated by the following examples.

*Example 1:*

5.9 g of zinc stearate (zinc precursor for core), 136 g of N-dodecyl-N'-phenylthiourea (sulfur precursor for core), 2.07 g of N,N,N'-tricyclohexylselenourea (selenium precursor for core) and 120 mL of octadecene are mixed in a round bottom flask. After degassing under vacuum, the mixture is heated to 260°C for 15 min under nitrogen flow and magnetic stirring, to synthesize the zinc chalcogenide core. Then the mixture is cooled down to room temperature and 10.6 g of zinc stearate (zinc precursor for shell) are introduced into the flask. The mixture is then heated to 280°C. During temperature increase, 20 mL of 1.0 M trioctylphosphine sulfide (TOP-S, sulfur precursor for shell) is added dropwise, leading to the formation of a shell. After injection of TOP-S and 2 hours at 280°C, the mixture is cooled down to room temperature. The obtained core-shell nanoparticles were purified by adding isopropanol and centrifugation twice. The core-shell nanoparticles are finally dispersed in heptane. The core-shell nanoparticles have a core of mean diameter 3.0 nm and formula $ZnSe_xS_{(1-x)}$ with x about 0.94 and a shell of mean thickness 1.3 nm.

Absorbance of a dispersion of 2 mg of core-shell nanospheres in 3 mL heptane is measured with a Jasco V770 UV-visible-NIR instrument with increments of 1 nm, and reported on figure 2 (solid line). Absorbance shows a maximum $A_{max}=0.41$ at 403 nm (shown by an arrow). Absorbance decreases by more than two orders of magnitude in 20 nm: absorption is almost complete at 403 nm and almost no absorption is observed at 426.5 nm.

40 mg of core-shell nanospheres are encapsulated in a mixed oxide of Aluminium (40 at%) and Zirconium (60 at%) with a loading charge of nanospheres in composite particles of 15 wt% according to the process disclosed in WO2018/220165. Then composite particles are introduced in a mixture of water, glyceryl stearate laurethyl 23, PEG-20 stearate and C10-15 alkyl lactate in proportions 15:2:1:1 - hereafter medium A - with a weight fraction of 12%.

*Example 2:*

Example 1 is reproduced at a larger scale in a 10 L reactor. The nanospheres obtained have a core of mean diameter 2.8 nm and formula $ZnSe_xS_{(1-x)}$ with x about 0.94 and a shell of mean thickness 1.2 nm.

Absorbance of a dispersion of 2 mg of core-shell nanospheres in 3 mL heptane is measured with a Jasco V770 UV-visible-NIR instrument with increments of 1 nm, and reported on figure 2 (double line). Absorbance shows a maximum $A_{max}$ =0.42 at 383 nm (shown by an arrow).

40 mg of core-shell nanospheres are encapsulated in a mixed oxide of Aluminium (60 at%) and Zirconium (40 at%) with a loading charge of nanospheres in composite particles of 10 wt% according to the process disclosed in WO2018/220165. Then composite particles are introduced in medium A with a weight fraction of 12%.

*Example 3:*

Example 1 is reproduced, but no shell is deposited on the nanoparticules. Finally Zinc chalcogenide nanospheres of formula $ZnSe_xS_{(1-x)}$ with x about 0.94 are obtained, with a mean diameter of 3 nm.

Absorbance of a dispersion of 2 mg of nanospheres in 3 mL heptane is measured with a Jasco V770 UV-visible-NIR instrument with increments of 1 nm, and reported on figure 2 (dotted line). Absorbance shows a maximum $A_{max}$ =0.49 at 370 nm (shown by an arrow).

40 mg of nanospheres - in alkaline water - are encapsulated in alumina with a loading charge of nanospheres in composite particles of 10 wt% according to the process disclosed in WO2018/220165. Then composite particles are introduced in a mixture of water (7), sorbitol (0.5), triethanolamine (0.1), benzyl alcohol (0.05), lanolin (0.45), cocoa butter (0.2), glyceryl stearate (0.3) and stearic acid (0.2) - hereafter medium B - with a weight fraction of 10%.

*Examples 4-10:*

The following table 1 lists compositions of composite particles and personal care compositions for examples 4-10.

In examples 6 to 8, zinc chalcogenide nanoparticles used are the same as those used in example 2: nanospheres have a core of mean diameter 2.8 nm and formula $ZnSe_xS_{(1-x)}$ with x about 0.94 and a shell of mean thickness 1.2 nm.

Nanosized ZnO is available from US Research Nanomaterials, Inc., as 18 nm or 10 nm-30 nm crystalline spherical nanoparticles - product reference 7712 or 355.

Nanosized $TiO_2$ is available US Research Nanomaterials, Inc., as 15 nm or 18 nm crystalline - anatase - spherical nanoparticles, for instance in isopropanol nanoparticles - product reference 269 or 3572.

All personal care compositions present satisfying SPF performance, as well as good transparency after application, while using only inorganic UV absorbers under the form of "nano-free" compounds - *i.e.*, without particles of size below 100 nm.

Table 1

| Ex. # | nanoparticles | | | composite particle | | composition | |
|---|---|---|---|---|---|---|---|
| | Zn chalcogenide (not ZnO) | ZnO | $TiO_2$ | matrix | Loading charge (wt%) | medium | composite particles weight fraction (wt%) |
| 1 | $ZnSe_xS_{(1-x)}$/ZnS | - | - | $Al_{2/9}Zr_{1/3}O$ | 15 | A | 12 |
| 2 | $ZnSe_xS_{(1-x)}$/ZnS | - | - | $Al_{6/17}Zr_{4/17}O$ | 10 | A | 12 |
| 3 | $ZnSe_xS_{(1-x)}$ | - | - | $Al_2O_3$ | 10 | B | 10 |
| 4 | - | 18 nm | | $Al_{2/5}Zr_{1/5}O$ | 15 | A | 8 |
| 5 | - | - | 15 nm | $ZrO_2$ | 10 | A | 8 |
| 6 | $ZnSe_xS_{(1-x)}$/ZnS | 18 nm | - | $Al_2O_3$ | 12 | B | 10 |
| 7 | $ZnSe_xS_{(1-x)}$/ZnS | - | 15 nm | $Al_{2/9}Zr_{1/3}O$ | 15 | B | 10 |

(continued)

| Ex. # | nanoparticles | | | composite particle | | composition | |
|---|---|---|---|---|---|---|---|
| | Zn chalcogenide (not ZnO) | ZnO | TiO$_2$ | matrix | Loading charge (wt%) | medium | composite particles weight fraction (wt%) |
| 8 | ZnSe$_x$S$_{(1-x)}$/ZnS | 18 nm | 15 nm | Al$_{6/17}$Zr$_{4/17}$O | 18 | B | 10 |
| 9 | - | 10-30 nm | - | Si$_{1/3}$Zr$_{1/6}$O | 12 | A | 8 |
| 10 | - | - | 18 nm | Si$_{4/10}$Zr$_{1/10}$O | 12 | A | 8 |

**Claims**

1. A personal care composition comprising

   • a cosmetically acceptable medium;
   • composite particles having a mean size in a range from 100 nm to 10 $\mu$m, said composite particles comprising:

      o nanoparticles having a size of less than 50 nm along at least one dimension; and comprising at least one of zinc chalcogenide nanoparticles or titanium oxide nanoparticles; and
      o a metal oxide matrix in which the nanoparticles are embedded.

2. The personal care composition according to claim **1,** wherein the nanoparticles comprise zinc chalcogenide nanoparticles comprising homostructured zinc chalcogenide nanoparticles having a composition defined by formula (I):

   ZnX          (I);

   where X is selected from sulfur, selenium, tellurium or mixtures thereof, preferably X is a mixture comprising more than 60 at% of selenium.

3. The personal care composition according to claims **1** or **2,** wherein the nanoparticles comprise zinc chalcogenide nanoparticles comprising heterostructured zinc chalcogenide nanoparticles having a composition defined by formula (II):

   ZnX$_1$/ZnX$_2$          (II);

   where X$_1$ is selected from oxygen, sulfur, selenium, tellurium and mixtures thereof; X$_2$ is selected from oxygen, sulfur, selenium, tellurium and mixtures thereof; and X$_1$ and X$_2$ are different.

4. The personal care composition according to claim **2** or **3,** wherein the zinc chalcogenide nanoparticles have local maximum absorbance of highest wavelength in a range from 350 nm to 450 nm.

5. The personal care composition according to any one of claims **2** to **4,** further comprising composite particles having a mean size in a range from 100 nm to 10 $\mu$m, said composite particles comprising titania nanoparticles embedded in the metal oxide matrix, and said titania nanoparticles having a size of less than 50 nm along at least one dimension, preferably less than 20 nm along at least one dimension.

6. The personal care composition according to any one of claims **2** to **5,** further comprising composite particles having a mean size in a range from 100 nm to 10 $\mu$m, said composite particles comprising zinc oxide nanoparticles embedded in the metal oxide matrix, and said zinc oxide nanoparticles having a size of less than 50 nm along at least one dimension, preferably less than 35 nm along at least one dimension.

7. The personal care composition according to any one of claims **1** to **6,** wherein the composite particles comprise:

   • zinc chalcogenide nanoparticles that are not zinc oxide nanoparticles and having a size of less than 20 nm along at least one dimension; and
• at least one of:

    o titania nanoparticles having a size of less than 20 nm along at least one dimension ;

    o zinc oxide nanoparticles having a size of less than 35 nm along at least one dimension.

8. The personal care composition according to any one of claims **5** to **7,** wherein the atomic ratio defined by the amount of zinc element in zinc chalcogenide nanoparticles that are not zinc oxide nanoparticles divided by the amount of zinc and titanium elements in zinc oxide nanoparticles and titanium oxide nanoparticles is in a range from 0.2 to 5.

9. The personal care composition according to any one of claims **1** to **8,** wherein the loading charge of the nanoparticles in the composite particle is at least 1%, preferably at least 2.5%, more preferably at least 5%, said loading charge being the mass ratio between the mass of nanoparticles comprised in a composite particle and the mass of said composite particle.

10. The personal care composition according to any one of claims **2** to **8,** wherein the loading charge of the zinc chalcogenide nanoparticles that are not zinc oxide nanoparticles in the composite particle is at least 1%, preferably at least 2.5%, more preferably at least 5%, said loading charge being the mass ratio between the mass of zinc chalcogenide nanoparticles that are not zinc oxide nanoparticles comprised in a composite particle and the mass of said composite particle.

11. The personal care composition according to claim **1,** wherein the composite particles comprise:

    • titania nanoparticles having a size of less than 20 nm along at least one dimension ; and

    • zinc oxide nanoparticles having a size of less than 35 nm along at least one dimension.

12. The personal care composition according to claim **11,** wherein the loading charge of the nanoparticles in the composite particle is at least 1%, preferably at least 2.5%, more preferably at least 5%, said loading charge being the mass ratio between the mass of nanoparticles comprised in a composite particle and the mass of said composite particle.

13. The personal care composition according to any one of claims **1** to **12,** wherein the metal oxide matrix is selected from the group consisting of $SiO_2$, $Al_2O_3$, $ZrO_2$, $HfO_2$, $GeO_2$, $SnO_2$, ZnO or a mixture thereof, preferably, the metal oxide matrix is alumina, $Al_yZr_zO$ with $\frac{3}{2}y + 2z = 1$ or $Si_pZr_qO$ with $p + q = \frac{1}{2}$.

14. The personal care composition according to any one of claims **1** to **13,** wherein the personal care composition substantially absorbs ultraviolet light.

15. The personal care composition according to any one of claims **1** to **14,** wherein refractive index of composite particles and refractive index of cosmetically-acceptable medium are matched, preferably the difference between the refractive index of composite particles and the refractive index of cosmetically-acceptable medium is less than 0.7, preferably less than 0.5, more preferably less than 0.4.

**FIG. 1**

**FIG. 2**

**FIG. 3**

| | Europäisches Patentamt European Patent Office Office européen des brevets | **EUROPEAN SEARCH REPORT** | **Application Number** EP 24 30 6693 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2009/126722 A1 (KOBO PRODUCTS INC [US]; SCHLOSSMAN DAVID [US] ET AL.) 15 October 2009 (2009-10-15) | 1,9, 13-15 | INV. A61K8/02 A61K8/26 |
| Y | * page 1, paragraph [0001] * * pages 21-22; example 1 * * pages 23-25; examples 3, 4 * * claims 1-4, 7, 10,12, 18, 22, 23, 78 * ----- | 5-8,11, 12 | A61K8/27 A61K8/28 A61K8/29 A61Q17/04 |
| X | US 2017/112731 A1 (GERSHON TALIA S [US] ET AL) 27 April 2017 (2017-04-27) | 1,2,4,9, 10,13-15 | |
| Y | * page 1, paragraphs [0008], [0012] * * page 2, paragraph [0014] * * claims 1-3, 5, 11, 13 * * figures 1, 2 * ----- | 5-8,11, 12 | |
| X | EP 1 230 907 A1 (SHISEIDO CO LTD [JP]) 14 August 2002 (2002-08-14) * page 2, paragraph [0005]-[0006]; examples 1-3, 5 * * page 4; examples 1-1, 1-2, 1-3 * * page 5; examples 3, 5 * * page 11; example 9 * * pages 12-13; example 13 * * page 13, paragraph [0076] * * claims 1-4, 7, 8 * ----- | 1,9, 13-15 | TECHNICAL FIELDS SEARCHED (IPC) A61K A61Q |
| X | EP 3 395 322 A1 (NIPPON SHEET GLASS CO LTD [JP]) 31 October 2018 (2018-10-31) * page 2, paragraphs [0008], [0010], [0012] * * page 6, paragraph [0041] * * pages 5-6; example 1 * * claims 1-4 * * figures 1-2 * ----- -/-- | 1,9, 13-15 | |

The present search report has been drawn up for all claims

| Place of search | Date of completion of the search | Examiner |
|---|---|---|
| The Hague | 23 March 2025 | Cielen, Elsie |

EPO FORM 1503 03.82 (P04C01)

Europäisches
Patentamt

European
Patent Office

Office européen
des brevets

**EUROPEAN SEARCH REPORT**

Application Number

EP 24 30 6693

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2012/104163 A1 (OREAL [FR]; GRARE CECILE [FR]; PHILIPPON CELINE [FR]) 9 August 2012 (2012-08-09) <br> * page 1, lines 5-10 * <br> * page 2, lines 18-20 * <br> * page 3, lines 13-19 * <br> * page 5, lines 4-8, 33-36 * <br> * pages 17-18; example 1 * <br> * claims 1, 3, 7, 9 * | 1,9, 13-15 | |
| A | WO 2013/072200 A2 (UNILEVER NV [NL]; UNILEVER PLC [GB] ET AL.) 23 May 2013 (2013-05-23) <br> * Reference 6 under the table; page 23 * | 1 | |
| X | WO 2023/139038 A1 (BASF SE [DE]) 27 July 2023 (2023-07-27) <br> * pages 60-62; examples 1-4 * <br> * pages 62-63; examples experiment 2-3 * <br> * claims 1, 5, 11, 26 * | 1,9, 13-15 | |
| A | US 2022/411695 A1 (NOJIMA YOSHIHIRO [JP] ET AL) 29 December 2022 (2022-12-29) <br> * claims 10, 12 * | 2-4,7,8, 10 | TECHNICAL FIELDS SEARCHED (IPC) |
| T | WO 2025/012330 A1 (NEXDOT [FR]) 16 January 2025 (2025-01-16) <br> * page 4, paragraph [0016] * <br> * claims 1, 4-8, 10 * | 1-15 | |

The present search report has been drawn up for all claims

| Place of search | Date of completion of the search | Examiner |
|---|---|---|
| The Hague | 23 March 2025 | Cielen, Elsie |

EPO FORM 1503 03.82 (P04C01)

## ANNEX TO THE EUROPEAN SEARCH REPORT
## ON EUROPEAN PATENT APPLICATION NO.

EP 24 30 6693

This annex lists the patent family members relating to the patent documents cited in the above-mentioned European search report.
The members are as contained in the European Patent Office EDP file on
The European Patent Office is in no way liable for these particulars which are merely given for the purpose of information.

23-03-2025

| Patent document cited in search report | | | Publication date | Patent family member(s) | | Publication date |
|---|---|---|---|---|---|---|
| WO 2009126722 | A1 | | 15-10-2009 | US | 2009258230 A1 | 15-10-2009 |
| | | | | US | 2014308221 A1 | 16-10-2014 |
| | | | | WO | 2009126722 A1 | 15-10-2009 |
| US 2017112731 | A1 | | 27-04-2017 | NONE | | |
| EP 1230907 | A1 | | 14-08-2002 | AU | 9026401 A | 02-04-2002 |
| | | | | EP | 1230907 A1 | 14-08-2002 |
| | | | | JP | 4755803 B2 | 24-08-2011 |
| | | | | JP | WO2002024153 A1 | 29-01-2004 |
| | | | | KR | 20020060230 A | 16-07-2002 |
| | | | | TW | I292322 B | 11-01-2008 |
| | | | | US | 2003068345 A1 | 10-04-2003 |
| | | | | WO | 0224153 A1 | 28-03-2002 |
| EP 3395322 | A1 | | 31-10-2018 | CN | 108430437 A | 21-08-2018 |
| | | | | EP | 3395322 A1 | 31-10-2018 |
| | | | | JP | 2017114826 A | 29-06-2017 |
| | | | | US | 2019000726 A1 | 03-01-2019 |
| | | | | WO | 2017110053 A1 | 29-06-2017 |
| WO 2012104163 | A1 | | 09-08-2012 | FR | 2971148 A1 | 10-08-2012 |
| | | | | WO | 2012104163 A1 | 09-08-2012 |
| WO 2013072200 | A2 | | 23-05-2013 | NONE | | |
| WO 2023139038 | A1 | | 27-07-2023 | CN | 118574599 A | 30-08-2024 |
| | | | | EP | 4465950 A1 | 27-11-2024 |
| | | | | JP | 2025502362 A | 24-01-2025 |
| | | | | KR | 20240136389 A | 13-09-2024 |
| | | | | US | 2025090429 A1 | 20-03-2025 |
| | | | | WO | 2023139038 A1 | 27-07-2023 |
| US 2022411695 | A1 | | 29-12-2022 | CN | 114746363 A | 12-07-2022 |
| | | | | JP | 7273992 B2 | 15-05-2023 |
| | | | | JP | WO2021111777 A1 | 10-06-2021 |
| | | | | KR | 20220110486 A | 08-08-2022 |
| | | | | TW | 202128953 A | 01-08-2021 |
| | | | | US | 2022411695 A1 | 29-12-2022 |
| | | | | WO | 2021111777 A1 | 10-06-2021 |
| WO 2025012330 | A1 | | 16-01-2025 | EP | 4491694 A1 | 15-01-2025 |
| | | | | WO | 2025012330 A1 | 16-01-2025 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018220165 A